# EUROPEAN PATENT SPECIFICATION

(11) **EP 3 960 107 B1**
(45) Date of publication and mention of the grant of the patent: **07.02.2024**
(21) Application number: 21193327.0
(22) Date of filing: 26.08.2021
(51) Int. Cl.: A61B 18/14, A61B 18/00, A61B 18/12

(54) **PROXIMAL ELECTRODE COOLING**
KÜHLUNG VON PROXIMALEN ELEKTRODEN
REFROIDISSEMENT D'ÉLECTRODE PROXIMALE

(30) Priority: 28.08.2020 US 202017006667
(43) Date of publication of application: 02.03.2022
(73) Proprietor: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL); BEECKLER, Christopher Thomas, Irvine, 92618 (US)
(74) Representative: Carpmaels & Ransford LLP

(56) References cited:
- US-A1- 2012 232 326
- US-A1- 2013 137 980
- US-A1- 2017 252 103
- US-A1- 2017 312 026

## Description

### FIELD OF THE INVENTION

The present invention relates to medical devices, and in particular, but not exclusively to, ablation catheters.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/006455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied through the tip electrode(s) of the ablating catheter, and current flows through the media that surrounds it, i.e., blood and tissue, between the tip electrode(s) and an indifferent electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Irreversible electroporation (IRE) applies short electrical pulses that generate high enough electrical fields (typically greater than 450 Volts per centimeter) to irreversibly damage the cells. Non-thermal IRE may be used in treating different types of tumors and other unwanted tissue without causing thermal damage to surrounding tissue. Small electrodes are placed in proximity to target tissue to apply short electrical pulses. The pulses increase the resting transmembrane potential, so that nanopores form in the plasma membrane. When the electricity applied to the tissue is above the electric field threshold of the target tissue, the cells become permanently permeable from the formation of nanopores. As a result, the cells are unable to repair the damage and die due to a loss of homeostasis and the cells typically die by apoptosis.

IRE may be used for cardiac ablation as an alternative to other cardiac ablation techniques, e.g., radio-frequency (RF) cardiac ablation. IRE cardiac ablation is sometimes referred to as Pulse Field Ablation (PFA). As IRE is generally a low thermal technique, IRE may reduce the risk of collateral cell damage that is present with the other techniques, e.g., in RF cardiac ablation.

US Patent 9,011,430 to Habib describes a device and method suitable for remodeling the internal surface of a hollow vessel at least partially occluded by a mass. The device comprises an elongate body having a distal end and a proximal end, the distal end comprising a tip portion located at the distal terminus of the body, and at least one heating element located proximally to the tip portion within the distal end. The at least one heating element is configured to be greater in dimension proximally than distally and thereby tapers towards the distal end. Furthermore, the at least one heating element is arranged so that it can be deployed outwardly from the body of the device and in so doing exert an expansion force on the hollow vessel.

US Patent Publication 2020/0038103 of Pappone, et al., describes a fluid delivery balloon ablation catheter configured to allow uniform fluid distribution through each electrode by varying the diameter of the main lumen. The catheter comprises an elongated tubular catheter body having a distal end, a proximal end, and a lumen extending longitudinally within the catheter body. A number of elution holes are provided in the catheter tip region, and these holes are in fluid communication with the lumen through ducts. As such, cooling fluid is delivered from the pump, through the lumen through the ducts, and out of the holes to the environment outside of the catheter. The main lumen has at least one tapered flow constrictor to restrict flow of fluid towards the distal region of the lumen. The catheter has multiple half-dome balloons, with the distal balloon being the smallest, and the proximal balloon being the largest balloon.

US Patent Publication 2020/179045 of Engelman, et al., describes apparatuses and methods for treating a heart failure patient by ablating a nerve of the thoracic splanchnic sympathetic nervous system to increase venous capacitance and reduce pulmonary blood pressure.

US Patent 10,524,859 to Vrba, et al., describes systems, devices and methods for modulating targeted nerve fibers (e.g., hepatic neuromodulation) or other tissue. Systems, devices and methods for cooling energy delivery members are also provided. The systems may be configured to access tortuous anatomy of adjacent hepatic vasculature. The systems may be configured to target nerves surrounding (e.g., within adventitia of or within perivascular space of) an artery or other blood vessel, such as the common hepatic artery.

US Patent Publication 2016/0074112 of Himmelstein describes an ablation device for denervation including a catheter delivery mechanism including an elongated tube with a distal end and a proximal end, the distal end being insertable within a body lumen at a target nerve region. A guide wire, at least one radiofrequency electrode, a plurality of positioning elements, and a plurality of pressing elements initially located within the tube. The electrode being deployable from the tube at the target nerve region and forming a ring-shaped structure adjacent the distal tube end. The positioning elements being deployable from the tube at the target nerve region from a position of the tube further distal than the electrode. The pressing elements being deployable from the tube more proximal than the electrode for use in pressing the deployed electrode against tissue to be ablated.

International Patent Publication WO2015200518 of Apama Medical Inc. describes tissue ablation devices, systems, and methods for monitoring or analyzing one or more aspects of tissue ablation. The disclosure includes ablation catheters comprising an elongate shaft; an inflatable balloon carried by a distal region of the shaft; a flexible circuit, including a conductor in electrical communication with an ablation electrode, disposed outside of and carried by an outer surface of the inflatable balloon; and an ultrasound monitoring member, configured for use in monitoring at least one aspect of tissue ablation with the ablation electrode. US 2012/232326 A1 discloses a catheter with an elongate body having a distal end and a proximal end, the distal end comprising a tip portion located at the distal terminus of the body, and at least one heating element located proximally to the tip portion within the distal end.

### SUMMARY

The invention provides a medical system according to claim 1. Further embodiments of the invention are defined in the dependent claims.

### BRIEF DESCRIPTION OF THE DRAWINGS

The present invention will be understood from the following detailed description, taken in conjunction with the drawings in which:
Fig. 1 is a schematic view of a medical system constructed and operative in accordance with an embodiment of the present invention;
Fig. 2 is a schematic view of a catheter in a deployed form constructed and operative in accordance with an embodiment of the present invention;
Fig. 3 is a schematic view of the distal end of the catheter of Fig. 2 in a collapsed form;
Fig. 4A is a cross-sectional view of the distal end of the catheter of Fig. 2;
Fig. 4B is a more detailed cross-sectional view of the distal end of the catheter inside block B of Fig. 4A;
Fig. 5A is a cross-sectional view of the catheter of Fig. 2 along line A:A;
Fig. 5B is a cross-sectional view of the catheter of Fig. 2 along line B:B;
Fig. 6 is a schematic view of an alternative exemplary catheter.

### DESCRIPTION OF EXAMPLE EMBODIMENTS

### OVERVIEW

A balloon catheter or another catheter with an expandable distal end assembly such as a basket catheter may include electrodes on the distal end assembly that may be used for ablation. The catheter is inserted into a body part (e.g., a heart chamber) of a living subject and an ablation current is applied between catheter electrodes in order to ablate tissue of the body part.

A return electrode may be used so that the ablation current is applied between one or more of the distal end assembly electrodes and a return electrode. If the ablation current is applied between electrodes on the distal end assembly, or between one or more electrodes of the distal end assembly and a return electrode placed in the middle of the distal end assembly, the ablation current may avoid travelling through the tissue thereby reducing the efficacy of the ablation current. Placing the return electrode proximal to the expandable distal end assembly helps prevent the ablation current from travelling inside the distal end assembly. However, due to the concentration of the ablation energy at the proximal return electrode, the proximal return electrode may overheat or cause charring of tissue.

Embodiments of the present invention solve the above problems by providing an irrigated proximal electrode, which is placed at a distal end of a deflectable element of a catheter, proximally to an expandable distal end assembly such as a balloon or basket assembly including electrodes. The proximal electrode extends circumferentially around the deflectable element, and includes irrigation holes through which to irrigate the body part to prevent overheating and charring.

An irrigation tube placed in the deflectable element is in fluid communication with the irrigation holes of the proximal electrode. The irrigation holes are generally placed radially around, and longitudinally along, the proximal electrode.

The proximal electrode and the deflectable element define an annular hollow therebetween with the irrigation tube coupled to transfer irrigation fluid into the hollow so that the irrigation tube is in fluid communication with the irrigation holes via the hollow. In some embodiments a pump pumps irrigation fluid from an irrigation reservoir via the irrigation tube into the hollow and out of the irrigation holes.

In some embodiments, an ablation power generator is connected to the catheter, and applies an electrical signal between at least one of the electrodes and the proximal electrode.

In some embodiments, the expandable distal end assembly is also irrigated. A second irrigation tube may be placed in the deflectable element and delivers irrigation fluid into a region surrounded by the expandable distal end assembly. In some embodiments, the electrodes of the expandable distal end assembly (e.g., a balloon assembly) include irrigation holes that are in fluid communication with the second irrigation tube.

### SYSTEM DESCRIPTION

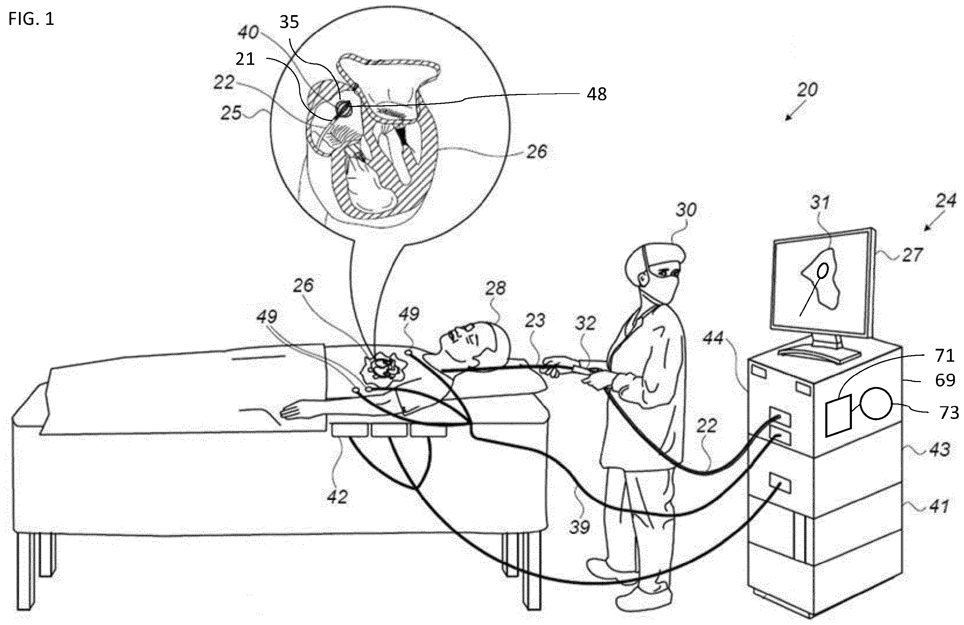

Reference is now made to Fig. 1, which is a schematic view of a medical system 20 constructed and operative in accordance with an embodiment of the present invention. The system 20 includes a catheter 40 configured to be inserted into a body part of a living subject (e.g., a patient 28). A physician 30 navigates the catheter 40 (for example, a basket catheter produced Biosense Webster, Inc. of Irvine, CA, USA), to a target location in a heart 26 of the patient 28, by manipulating an elongated deflectable element 22 of the catheter 40, using a manipulator 32 near a proximal end of the catheter 40, and/or deflection from a sheath 23. In the pictured embodiment, physician 30 uses catheter 40 to perform electro-anatomical mapping of a cardiac chamber and ablation of cardiac tissue.

Catheter 40 includes an expandable distal end assembly 35 (e.g., a basket assembly), which is inserted in a folded configuration, through sheath 23, and only after the catheter 40 exits sheath 23 does the distal end assembly 35 regain its intended functional shape. By containing distal end assembly 35 in a folded configuration, sheath 23 also serves to minimize vascular trauma on its way to the target location.

Catheter 40 includes a plurality of electrodes 48 disposed on the expandable distal end assembly 35 for sensing electrical activity and/or applying ablation power to ablate tissue of the body part. The catheter 40 also includes a proximal electrode 21 disposed on the deflectable element 22 proximal to the expandable distal end assembly 35. Catheter 40 may incorporate a magnetic position sensor (not shown) at the distal edge of deflectable element 22 (i.e., at the proximal edge of the distal end assembly 35). Typically, although not necessarily, the magnetic sensor is a Single-Axis Sensor (SAS). A second magnetic sensor (not shown) may be included at any suitable position on the assembly 35. The second magnetic sensor may be a Triple-Axis Sensor (TAS) or a Dual-Axis Sensor (DAS), or a SAS by way of example only, based for example on sizing considerations. The magnetic sensors, the proximal electrode 21, and electrodes 48 disposed on the assembly 35 are connected by wires running through deflectable element 22 to various driver circuitries in a console 24.

In some embodiments, system 20 comprises a magnetic-sensing subsystem to estimate an ellipticity of the basket assembly 35 of catheter 40, as well as its elongation/retraction state, inside a cardiac chamber of heart 26 by estimating the elongation of the basket assembly 35 from the distance between the magnetic sensors. Patient 28 is placed in a magnetic field generated by a pad containing one or more magnetic field generator coils 42, which are driven by a unit 43. The magnetic fields generated by coil(s) 42 transmit alternating magnetic fields into a region where the body-part is located. The transmitted alternating magnetic fields generate signals in the magnetic sensors, which are indicative of position and/or direction. The generated signals are transmitted to console 24 and become corresponding electrical inputs to processing circuitry 41.

The method of position and/or direction sensing using external magnetic fields and magnetic sensors, is implemented in various medical applications, for example, in the CARTO^{®} system, produced by Biosense-Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

Processing circuitry 41, typically part of a general-purpose computer, is further connected via a suitable front end and interface circuits 44, to receive signals from body surface-electrodes 49. Processing circuitry 41 is connected to body surface-electrodes 49 by wires running through a cable 39 to the chest of patient 28.

In an embodiment, processing circuitry 41 renders to a display 27, a representation 31 of at least a part of the catheter 40 and a mapped body-part, responsively to computed position coordinates of the catheter 40.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

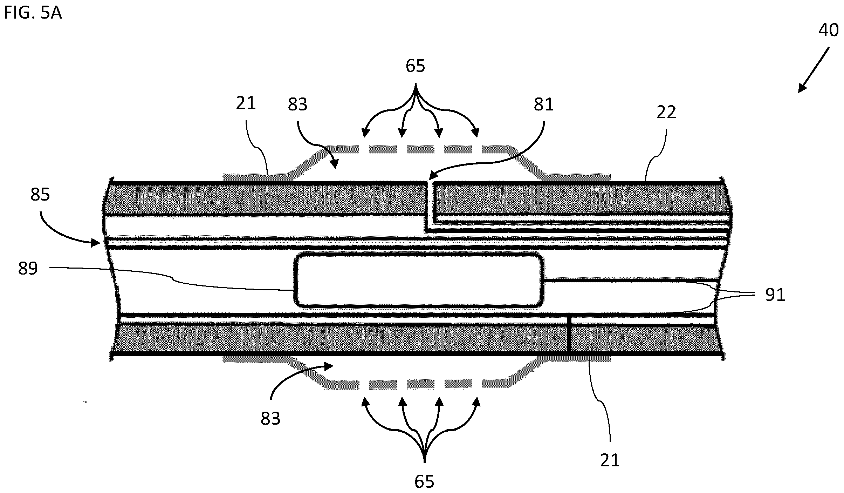

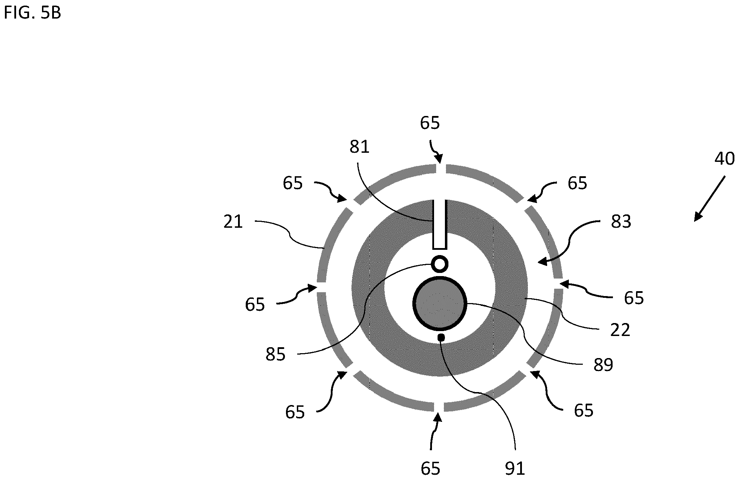

The medical system 20 may also include an ablation power generator 69 (such as an RF signal generator) configured to be connected to the catheter 40, and apply an electrical signal between one or more of the electrodes 48 and the proximal electrode 21. The medical system 20 may also include an irrigation reservoir 71 configured to store irrigation fluid, and a pump 73 configured to be connected to the irrigation reservoir 71 and the catheter 40, and to pump the irrigation fluid from the irrigation reservoir 71 via an irrigation tube through irrigation holes of the catheter 40 as described in more detail with reference to Figs. 5A and 5B.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description. The elements of system 20 and the methods described herein may be further applied, for example, to control an ablation of tissue of heart 26.

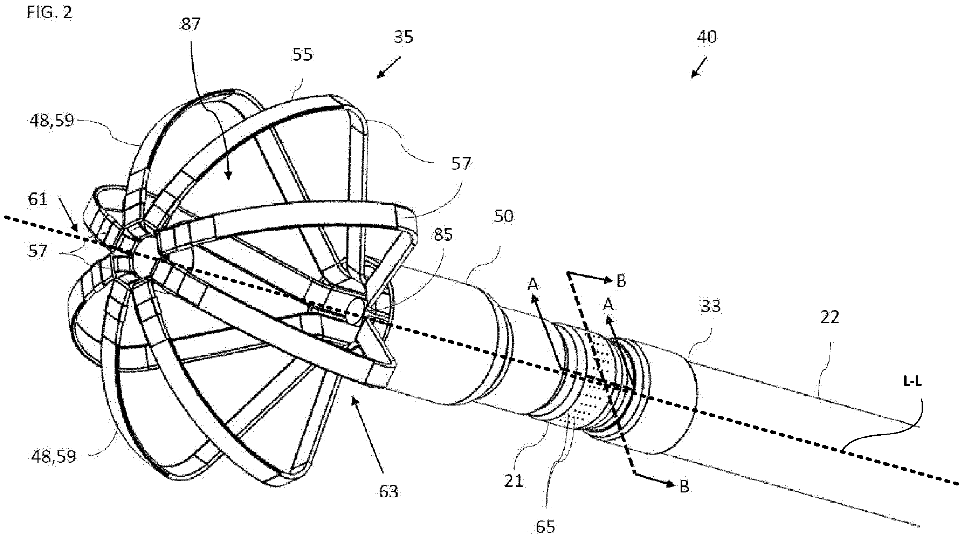

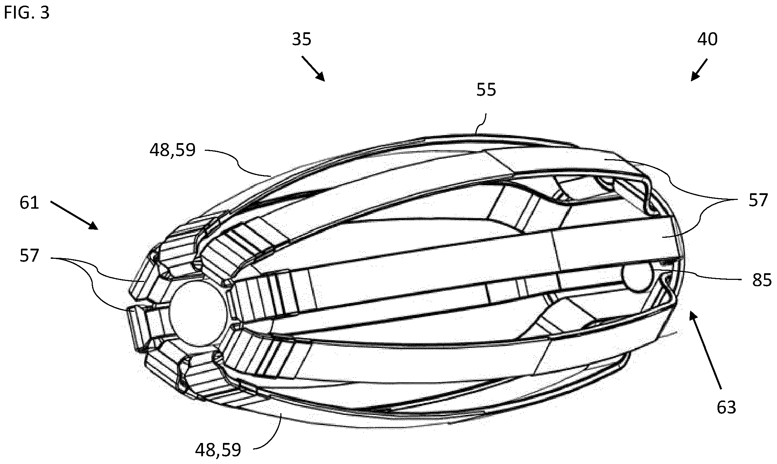

Reference is now made to Figs. 2 and 3. Fig, 2 is a schematic view of the catheter 40 in a deployed form constructed and operative in accordance with an embodiment of the present invention. Fig. 3 is a schematic view of the distal end of the catheter 40 of Fig. 2 in a collapsed form.

In Fig. 2, the catheter 40 extends along a longitudinal axis L-L from a proximal location (closest to an operator) to a distal location furthest away from the operator along the axis L-L. For example, portion 35 may be considered with respect to portion 33 as a "distal" portion while portion 33 may be considered a "proximal" portion. The catheter 40 is configured to be inserted into a body part (e.g., the heart 26 (Fig. 1)) of a living subject. The deflectable element 22 of the catheter 40 has a distal end 33. The deflectable element 22 may be produced from any suitable material, for example, polyurethane or polyether block amide. The assembly 35 is disposed distally to the deflectable element 22 and may be connected to the deflectable element 22 via a proximal coupling member 50 at the distal end 33. The proximal coupling member 50 typically comprises a hollow tube and may be formed from any suitable material, for example, but not limited to polycarbonate with or without glass filler, polyether ether ketone (PEEK) with or without glass filler, polyimide, polyamide, or Polyetherimide (PEI) with or without glass filler. The coupling member 50 may formed as an integral part of the deflectable element 22 or as part of the distal end assembly 35 or as a separate element which connects with the deflectable element 22 and the distal end assembly 35.

The assembly 35, which may include a basket assembly, may include multiple splines such as flexible strips 55 (only one labeled for the sake of simplicity) with the electrodes 48 disposed on the splines. In the embodiments of Figs. 2 and 3 each flexible strip 55 includes a single electrode 48 (only some labeled for the sake of simplicity). The assembly 35 may include any suitable number of electrodes 48 with multiple electrodes 48 per strip 55.

In the embodiment of Figs. 2 and 3, each flexible strip 55 is formed of Nitinol which is selectively covered with insulating material (for example, thermoplastic polymer resin shrink wrap (PET)) in the distal and proximal regions 57 (only some labeled for the sake of simplicity) of the flexible strips 55 leaving a central region 59 (only some labeled for the sake of simplicity) of the flexible strips 55 as an electrically active region to perform mapping and/or perform ablation or electroporation, by way of example. The structure of the assembly 35 may vary. For example, flexible strips 55 (or other splines) may include flexible printed circuit boards (PCBs), or a shape-memory alloy such as Nitinol. The electrically active region of each flexible strip 55 may be larger or smaller than that shown in Fig. 2, and/or more centrally or proximally disposed on each flexible strip 55.

Embodiments described herein refer mainly to a basket distal-end assembly 35, purely by way of example. In alternative embodiments, the disclosed techniques can be used with any other suitable type of distal-end assembly.

The distal end assembly 35 includes a distal portion 61, and a proximal portion 63, and is configured to expand from a collapsed form (shown in Fig. 3) to an expanded deployed form (shown in Fig. 2). The relaxed state of the distal end assembly 35 is the expanded deployed form shown in Fig. 2. The distal end assembly 35 is configured to collapse into the collapsed form when the catheter 40 is retracted in a sheath 23 (Fig. 1) and is configured to expand to the expanded deployed form when the catheter 40 is removed from the sheath 23. The relaxed shape of the distal end assembly 35 may be set by forming the flexible strips 55 from any suitable resilient material such as Nitinol or PEI. In some embodiments, the relaxed state of the expandable distal end assembly 35 may be the collapsed form, and the expandable distal end assembly 35 is expanded using a pull wire or element connected to the distal portion 61 and fed through a lumen in the deflectable element 22.

The proximal electrode 21 is disposed at the distal end 33 of the deflectable element 22 proximally to the expandable distal end assembly 35, and generally extends circumferentially around the deflectable element 22. The proximal electrode 21 includes irrigation holes 65 (only some labeled for the sake of simplicity) through which to irrigate the body part. The irrigation holes 65 are generally disposed radially around, and/or longitudinally along, the proximal electrode 21. The irrigation holes may have any suitable diameter, for example, in the range of 25 to 100 microns. The holes may be formed using any suitable technique, for example, laser drilling or electrical discharge machining (EDM). The proximal electrode 21 may include any suitable number of holes, for example, in the range of 4 to 100 holes. In one example, the proximal electrode 21 includes 5 proximally disposed holes and 5 distally disposed holes.

The ablation power generator 69 (Fig. 1) is configured to be connected to the catheter 40, and apply an electrical signal between at least one of the electrodes 48 and the proximal electrode 21. In some embodiments, the ablation power generator 69 is configured to apply the electrical signal between at least one of the electrodes 48 and the proximal electrode 21 to perform electroporation of tissue of the body part.

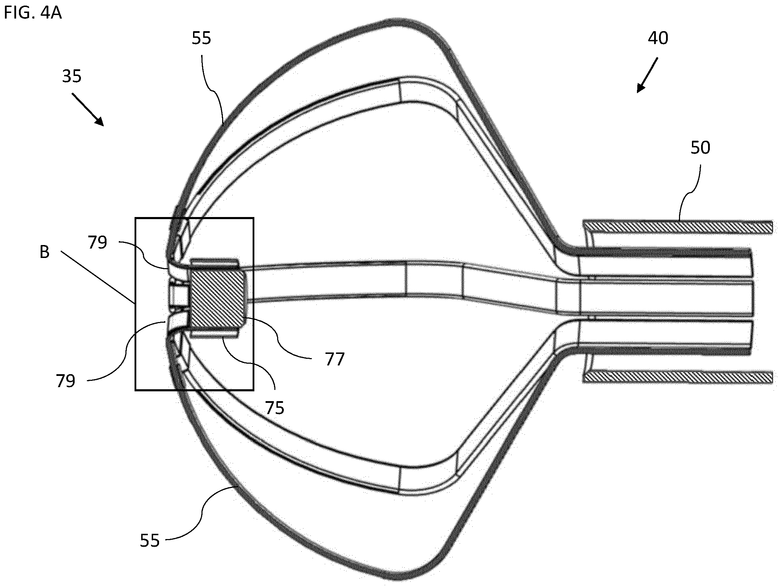

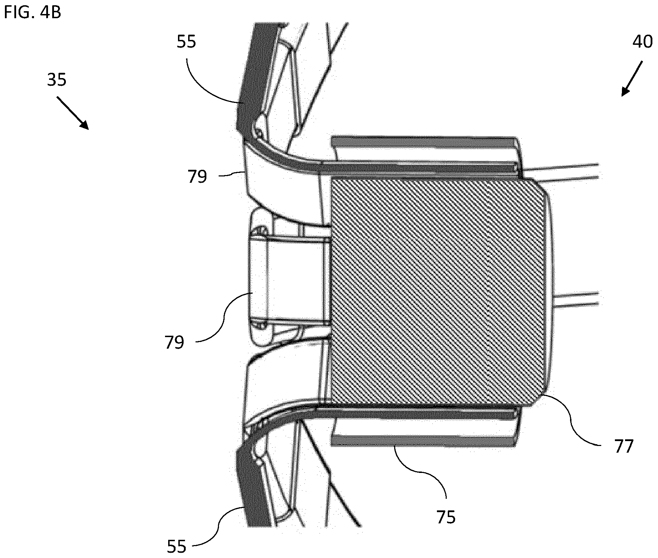

Reference is now made to Figs. 4A and 4B. Fig. 4A is a cross-sectional view of the distal end of the catheter 40 of Fig. 2. Fig. 4B is a more detailed cross-sectional view of the distal end of the catheter 40 inside block B of Fig. 4A.

The distal ends of the flexible strips 55 (only two labeled for the sake of simplicity) are folded over and connected to a distal connector 75, which in some embodiments is a tube (e.g., polymer tube) or slug (e.g., polymer slug). The distal connector 75 may be formed from any suitable material, for example, but not limited to polycarbonate with or without glass filler, PEEK with or without glass filler, or PEI with or without glass filler. In some embodiments, the flexible strips 55 may be connected to the distal connector 75 without being folded over so that when the distal end assembly 35 is collapsed the flexible strips 55 are approaching a flat formation along their length. The proximal ends of the flexible strips 55 are connected to the proximal coupling member 50. The flexible strips 55 may be connected to the distal connector 75 and the proximal coupling member 50 using a suitable adhesive, such as an epoxy adhesive.

In some embodiments, the catheter 40 includes a nose cap 77 inserted into the distal connector 75. The nose cap 77 may be used to help secure the flexible strips 55 to the distal connector 75. The nose cap 77 may be formed from any suitable material, for example, but not limited to polycarbonate with or without glass filler, PEEK with or without glass filler, or PEI with or without glass filler. The nose cap 77 may optionally be sized to provide a pressure fit against the flexible strips 55 to prevent the flexible strips 55 from being pulled away from the inner surface of the distal connector 75.

In some embodiments, the thickness of the distal portions of the flexible strips 55 may be reduced (compared to the rest of the flexible strips 55) to create hinges 79 (one hinge 79 per flexible strip 55) to allow the flexible strips 55 to bend sufficiently between the collapsed form and the deployed expanded form of the expandable distal end assembly 35. Only two of the hinges 79 are labeled for the sake of simplicity. The hinges 79 of the flexible strips 55 may be reinforced using a flexible material such as a yarn (not shown). The hinges 79 (including the yarn and covering layers) may have any suitable thickness, for example, in the range of 10 to 140 microns. The yarn may comprise any one or more of the following: an ultra-high-molecular-weight polyethylene yarn; or a yarn spun from a liquid-crystal polymer. The yarn may be any suitable linear density, for example, in a range between 25 denier and 250 denier.

Reference is now made to Figs. 5A and 5B. Fig 5A is a cross-sectional view of the catheter 40 of Fig. 2 along line A:A. Fig. 5B is a cross-sectional view of the catheter of Fig. 2 along line B:B.

Figs. 5A and 5B show the proximal electrode 21 which extends circumferentially around the deflectable element 22. The edges of the proximal electrode 21 may be connected to the deflectable element 22 using a suitable adhesive and/or using a covering such as a thermoplastic polymer resin shrink wrap. Figs. 5A and 5B show some of the irrigation holes 65 (only some labeled for the sake of simplicity) in the proximal electrode 21. The proximal electrode 21 may have any suitable width measured parallel to the direction of elongation of the deflectable element 22, for example, in the range of 2 and 10 mm.

The catheter 40 includes an irrigation tube 81 disposed in the deflectable element 22 and configured to be in fluid communication with the irrigation holes 65 of the proximal electrode 21. The pump 73 (Fig. 1) is configured to be connected to the irrigation reservoir 71 (Fig. 1) and the catheter 40, and to pump the irrigation fluid from the irrigation reservoir 71 through the irrigation holes 65 via the irrigation tube 81.

The inner surface of the proximal electrode 21 and the deflectable element 22 define an annular hollow 83 therebetween. The irrigation tube 81 is coupled to the annular hollow 83 to transfer the irrigation fluid into the hollow 83. The irrigation tube 81 is generally disposed on the other side of the annular hollow 83 to the irrigation holes 65. Therefore, the irrigation tube 81 is in fluid communication with the irrigation holes 65 via the hollow 83. The pump 73 (Fig. 1) is configured to pump the irrigation fluid from the irrigation reservoir 71 via the irrigation tube 81 into the hollow 83 and out of the irrigation holes 65. The collection of the irrigation fluid in the annular hollow 83 acts to cool the outer surface of the proximal electrode 21 and not just the portions close to the irrigation holes 65.

The catheter 40 may include another irrigation tube 85 disposed in the deflectable element 22 and configured to deliver irrigation fluid into a region 87 (Fig. 2) surrounded by the flexible strips 55 of the expandable distal end assembly 35. The irrigation tube 85 typically extends into the expandable distal end assembly 35 as shown in Figs. 2 and 3.

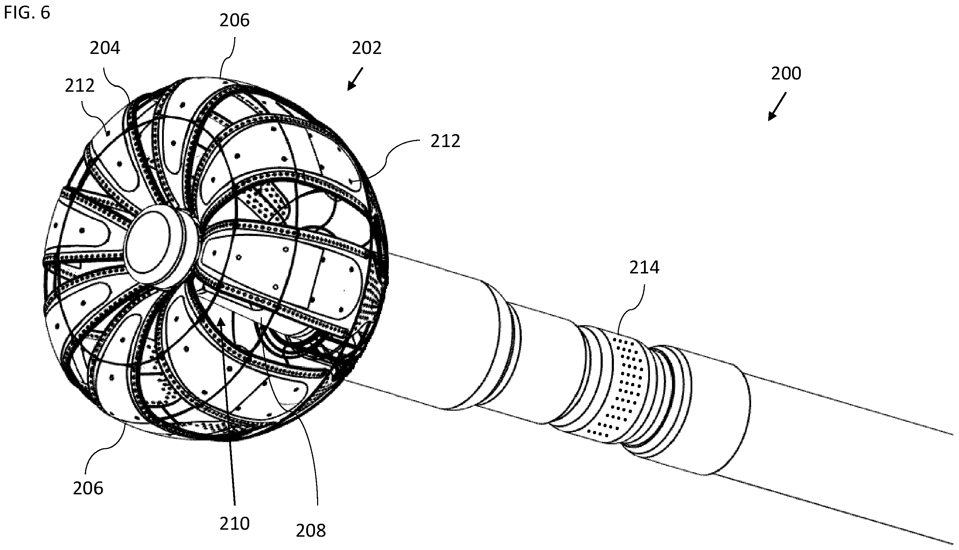

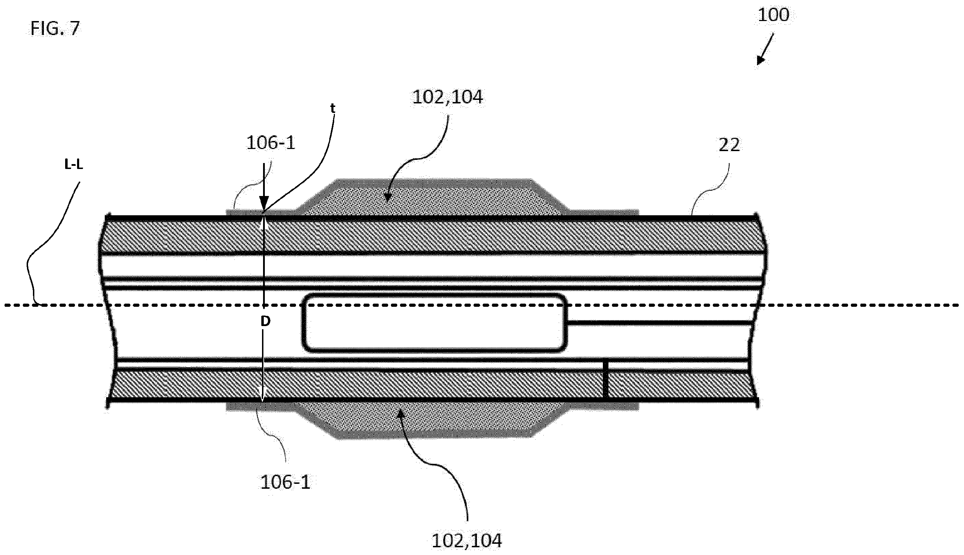

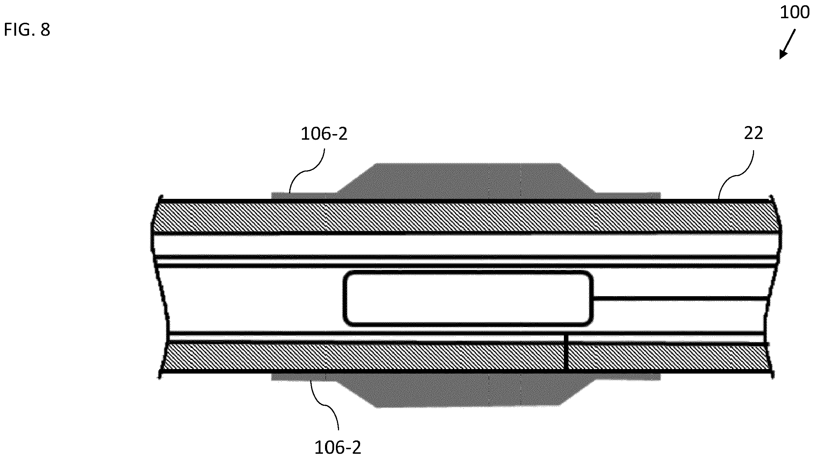

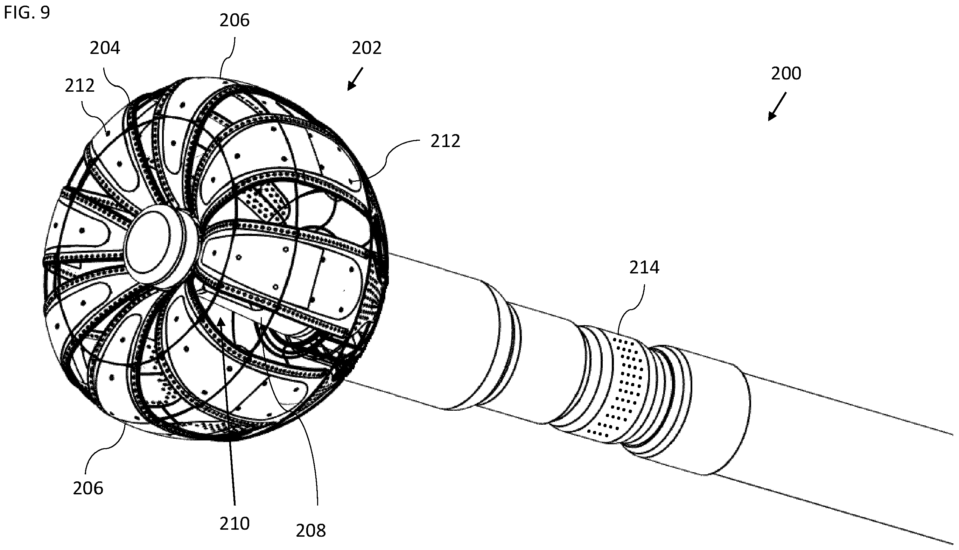

In some embodiments, the catheter 40 includes a position sensor 89 (such as a magnetic position sensor) disposed in the deflectable element 22. Figs. 5A and 5B also show wires 91 disposed therein connecting the electrodes 48, the proximal electrode 21, and the position sensor 89 with the proximal end of the catheter 40.Reference is now made to Fig. 6, which is a schematic view of a balloon catheter 200 in a deployed form constructed and operative in accordance with yet another alternative embodiment of the present invention. The catheter 200 is substantially the same as the catheter 40 of Fig. 2 except that the catheter 200 includes an expandable distal end assembly 202, which includes an inflatable balloon 204 with the electrodes 206 (only some labeled for the sake of simplicity) disposed thereon. The catheter 200 includes an irrigation tube 208 which is disposed in the deflectable element 22 and extends into a region 210 surrounded by the inflatable balloon 204. The electrodes 206 of the expandable distal end assembly 202 include irrigation holes 212 (only some labeled for the sake of simplicity) that are in fluid communication with the irrigation tube 208. The catheter 200 includes a proximal electrode 214 in substantially the same form as the proximal electrode 21 described with reference to Figs. 5A and 5B.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention is defined by the appended claims.

## Claims

1. A medical system comprising a catheter (40) configured to be inserted into
a body part of a living subject, and including:
a deflectable element (22) extending along a longitudinal axis from a proximal portion to a distal end;
an expandable distal end assembly (35) disposed proximate the distal end of the deflectable element (22), and comprising a plurality of electrodes (48) disposed about the longitudinal axis, and configured to expand from a collapsed form to an expanded deployed form;
a proximal electrode (21) disposed proximate the distal end of the deflectable element (22) adjacent to the expandable distal end assembly (35), and extending circumferentially around the deflectable element, and including irrigation holes (65) through which irrigation fluid is provided thereto;
an annular hollow (83) defined between the proximal electrode (21) and the deflectable element (22); and
an irrigation tube (81) disposed in the deflectable element, coupled to the annular hollow (83) to transfer irrigation fluid into the annular hollow (83), and configured to be in fluid communication with the irrigation holes of the proximal electrode via the annular hollow (83).

2. The system according to claim 1, wherein the irrigation holes are disposed radially around the proximal electrode.

3. The system according to claim 2, wherein the irrigation holes are disposed longitudinally along the longitudinal axis and radially about the proximal electrode.

4. The system according to claim 1, wherein the irrigation holes are disposed longitudinally along the proximal electrode.

5. The system according to claim 1, further comprising:
an ablation power generator (69) configured to be connected to the catheter, and apply an electrical signal between at least one of the electrodes and the proximal electrode;
an irrigation reservoir (71) configured to store irrigation fluid; and
a pump (73) configured to be connected to the irrigation reservoir and the catheter, and to pump the irrigation fluid from the irrigation reservoir through the irrigation holes via the irrigation tube.

6. The system according to claim 5, the pump being configured to pump the irrigation fluid from the irrigation reservoir via the irrigation tube into the annular hollow and out of the irrigation holes.

7. The system according to claim 1, further comprising another irrigation tube (85) disposed in the deflectable element and configured to deliver irrigation fluid into a region surrounded by the expandable distal end assembly.

8. The system according to claim 7, wherein the electrodes of the expandable distal end assembly include irrigation holes that are in fluid communication with the other irrigation tube.

## Patentansprüche

1. Medizinisches System, umfassend einen Katheter (40), der dazu ausgelegt ist, in einen Körperteil eines lebenden Subjekts eingesetzt zu werden, und aufweisend:
ein auslenkbares Element (22), das sich entlang einer Längsachse von einem proximalen Abschnitt zu einem distalen Ende erstreckt,
eine ausdehnbare distale Endanordnung (35), die in der Nähe des distalen Endes des auslenkbaren Elements (22) angeordnet ist, und eine Vielzahl von Elektroden (48) umfasst, die rund um die Längsachse angeordnet sind, und dazu ausgelegt ist,
sich aus einer zusammengefallenen Form in eine ausgedehnte eingesetzte Form auszudehnen,
eine proximale Elektrode (21), die in der Nähe des distalen Endes des auslenkbaren Elements (22) neben der ausdehnbaren distalen Endanordnung (35) angeordnet ist und sich in Umfangsrichtung um das auslenkbare Element erstreckt und Spüllöcher (65) aufweist, durch die Spülflüssigkeit dort bereitgestellt wird,
einen ringförmigen Hohlraum (83), der zwischen der proximalen Elektrode (21) und dem auslenkbaren Element (22) definiert ist, und
eine Spülröhre (81), die in dem auslenkbaren Element angeordnet ist, die mit dem ringförmigen Hohlraum (83) gekoppelt ist, um Spülflüssigkeit in den ringförmigen Hohlraum (83) zu übertragen, und die dazu ausgelegt ist, mit den Spüllöchern der proximalen Elektrode über den ringförmigen Hohlraum (83) in Flüssigkeitsverbindung zu sein.

2. System nach Anspruch 1, wobei die Spüllöcher radial um die proximale Elektrode angeordnet sind.

3. System nach Anspruch 2, wobei die Spüllöcher in Längsrichtung entlang der Längsachse und radial um die proximale Elektrode angeordnet sind.

4. System nach Anspruch 1, wobei die Spüllöcher in Längsrichtung entlang der proximalen Elektrode angeordnet sind.

5. System nach Anspruch 1, ferner umfassend:
einen Ablationsgenerator (69), der dazu ausgelegt ist, mit dem Katheter verbunden zu werden und ein elektrisches Signal zwischen mindestens einer der Elektroden und der proximalen Elektrode anzulegen,
einen Spülbehälter (71), der dazu ausgelegt ist, Spülflüssigkeit zu lagern, und
eine Pumpe (73), die dazu ausgelegt ist, mit dem Spülbehälter und dem Katheter verbunden zu werden und die Spülflüssigkeit aus dem Spülbehälter über die Spülröhre durch die Spüllöcher zu pumpen.

6. System nach Anspruch 5, wobei die Pumpe dazu ausgelegt ist, die Spülflüssigkeit aus dem Spülbehälter über die Spülröhre in den ringförmigen Hohlraum und aus den Spüllöchern zu pumpen.

7. System nach Anspruch 1, ferner umfassend eine andere Spülröhre (85), die in dem auslenkbaren Element angeordnet und dazu ausgelegt ist, Spülflüssigkeit in einen Bereich zu liefern, der von der ausdehnbaren distalen Endanordnung umgeben ist.

8. System nach Anspruch 7, wobei die Elektroden der ausdehnbaren distalen Endanordnung Spüllöcher aufweisen, die mit der anderen Spülröhre in Flüssigkeitsverbindung sind.

## Revendications

1. Système médical comprenant un cathéter (40) configuré pour être inséré dans une partie du corps d'un sujet vivant, et comprenant :
un élément élastique (22) s'étendant le long d'un axe longitudinal depuis une partie proximale jusqu'à une extrémité distale ;
un ensemble d'extrémité distale extensible (35) disposé à proximité de l'extrémité distale de l'élément élastique (22) et comprenant une pluralité d'électrodes (48) disposées autour de
l'axe longitudinal, et configuré pour s'étendre d'une forme repliée à une forme déployée élargie ;
une électrode proximale (21) disposée à proximité de l'extrémité distale de l'élément élastique (22), adjacente à l'ensemble d'extrémité distale extensible (35), et s'étendant circonférentiellement autour de l'élément élastique, et comprenant des trous d'irrigation (65) à travers lesquels un fluide d'irrigation est fourni à celui-ci ;
un creux annulaire (83) défini entre l'électrode proximale (21) et l'élément élastique (22) ; et
un tube d'irrigation (81) disposé dans l'élément élastique, couplé au creux annulaire (83) pour transférer le liquide d'irrigation dans le creux annulaire (83), et configuré pour être en communication fluidique avec les trous d'irrigation de l'électrode proximale par l'intermédiaire du creux annulaire (83).

2. Système selon la revendication 1, les trous d'irrigation étant disposés radialement autour de l'électrode proximale.

3. Système selon la revendication 2, les trous d'irrigation étant disposés longitudinalement le long de l'axe longitudinal et radialement autour de l'électrode proximale.

4. Système selon la revendication 1, les trous d'irrigation étant disposés longitudinalement le long de l'électrode proximale.

5. Système selon la revendication 1, comprenant en outre :
un générateur d'énergie d'ablation (69) configuré pour être raccordé au cathéter et appliquer un signal électrique entre au moins une des électrodes et l'électrode proximale ;
un réservoir d'irrigation (71) configuré pour stocker le liquide d'irrigation ; et
une pompe (73) configurée pour être raccordée au réservoir d'irrigation et au cathéter, et pour pomper le liquide d'irrigation du réservoir d'irrigation à travers les trous d'irrigation via le tube d'irrigation.

6. Système selon la revendication 5, la pompe étant configurée pour pomper le liquide d'irrigation du réservoir d'irrigation via le tube d'irrigation dans le creux annulaire et hors des trous d'irrigation.

7. Système selon la revendication 1, comprenant en outre un autre tube d'irrigation (85) disposé dans l'élément élastique et configuré pour délivrer le liquide d'irrigation dans une région entourée par l'ensemble d'extrémité distale extensible.

8. Système selon la revendication 7, les électrodes de l'ensemble d'extrémité distale extensible comprenant des trous d'irrigation qui sont en communication fluide avec l'autre tube d'irrigation.
